(19) Europäisches Patentamt
European Patent Office
Office européen des brevets

(11) EP 0 767 174 A1

# (12) EUROPÄISCHE PATENTANMELDUNG

(43) Veröffentlichungstag:
**09.04.1997 Patentblatt 1997/15**

(21) Anmeldenummer: **96118735.8**

(22) Anmeldetag: **12.11.1992**

(51) Int. Cl.[6]: **C07F 9/30**, A61K 31/66, C07F 9/58, C07F 9/60, C07F 9/655

(84) Benannte Vertragsstaaten:
**AT BE CH DE DK ES FR GB GR IE IT LI LU NL PT SE**

(30) Priorität: **21.11.1991 CH 3404/91**

(62) Dokumentnummer(n) der früheren Anmeldung(en) nach Art. 76 EPÜ:
**92810879.4 / 0 543 780**

(71) Anmelder: **CIBA-GEIGY AG**
**4002 Basel (CH)**

(72) Erfinder: **Mickel, Stuart John**
**4415 Lausen (CH)**

Bemerkungen:
Diese Anmeldung ist am 22 - 11 - 1996 als Teilanmeldung zu der unter INID-Kode 62 erwähnten Anmeldung eingereicht worden.

## (54) Neue Aminoalkanphosphinsäuren und ihre Salze

(57) Verbindungen der Formel I

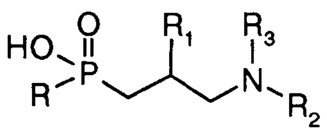

(I),

worin

a) R Diethoxymethyl, $R_1$ Wasserstoff und $R_2$ 2,6- oder 3,5-Dichlorbenzyl, Pyrid-3-ylmethyl, 1-(4-Methoxyphenyl)ethyl, 1-(4-Chlor-3-jod-phenyl)ethyl oder 1-(3-Chlor-4-jod-phenyl)ethyl oder $R_1$ Hydroxy und $R_2$ 3,4-Dichlorbenzyl, 1-(3-Chlor-4-jod-phenyl)ethyl, 1-(4-Chlor-3-jod-phenyl)ethyl oder 1-(3,4-Dichlorphenyl)ethyl und $R_3$ Wasserstoff bedeutet oder
b) R Cyclohexylmethyl, $R_1$ Wasserstoff und $R_2$ 3,5-Dichlorbenzyl, Chinolin-4-yl-methyl, 1-(3-Chlorphenyl)ethyl, oder 1-(3,4,5-Trimethoxyphenyl)ethyl oder $R_1$ Hydroxy und $R_2$ 3,4-Dimethylbenzyl, 3,4-Methylendioxybenzyl, 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)-ethyl, 1-(3,4-Methylendioxyphenyl)ethyl, 1-(3,5-Dimethoxy-phenyl)ethyl, 1-(3,4,5-Trimethoxyphenyl)ethyl,3-Phenylprop-2-yl, 2-(3,4-Dichlorphenyl)propyl 2-(3,4-Dichlorphenyl)propyl, 3-(3,4-Dichlorphenyl)prop-2-yl oder 3-Phenyl-3-hydroxy-prop-2-yl und $R_3$ Wasserstoff bedeutet oder R Cyclohexylmethyl, $R_1$ Wasserstoff, $R_2$ 4-Chlorbenzyl und $R_3$ Methyl bedeutet oder
c) R Cylohex-3-enylmethyl, $R_1$ (S)-Hydroxy, $R_2$ 1(S)-(3,4-Dichlorphenyl)ethyl und $R_3$ Wasserstoff bedeutet oder
d) R Benzyl, $R_1$ Hydroxy, $R_2$ $\alpha$-Cyclopropyl-3,4-dichlor-benzyl, 3,4,5-Trimethoxybenzyl, 1-(3,5-Dimethoxyphenyl)ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,4,5-Trimethoxyphenyl)ethyl, 3-Phenylprop-2-yl, 3-Phenyl-3-hydroxy-prop-2-yl, 1-, 2- oder 3-(3,4-Dichlorphenyl)propyl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet, oder
e) R 4-Chlorbenzyl, 4-Methylbenzyl, 4-Methoxybenzyl oder Cyclohex-3-enmethyl bedeutet und R1, R2 und R3 Wasserstoff darstellen,

EP 0 767 174 A1

und ihre pharmazeutisch verwendbaren Salze weisen GABAB-antagonistische Eigenschaften auf und können als nootrope Arzneimittelwirkstoffe verwendet werden.

**Beschreibung**

Die Erfindung betrifft Verbindungen der Formel I

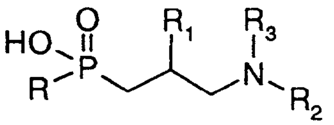

(I),

worin

a) R Diethoxymethyl, $R_1$ Wasserstoff und $R_2$ 2,6- oder 3,5-Dichlorbenzyl, Pyrid-3-ylmethyl, 1-(4-Methoxyphenyl)ethyl, 1-(4-Chlor-3-jod-phenyl)ethyl oder 1-(3-Chlor-4-jod-phenyl)ethyl oder $R_1$ Hydroxy und $R_2$ 3,4-Dichlorbenzyl, 1-(3-Chlor-4-jod-phenyl)ethyl, 1-(4-Chlor-3-jod-phenyl)ethyl oder 1-(3,4-Dichlorphenyl)ethyl und $R_3$ Wasserstoff bedeutet oder
b) R Cyclohexylmethyl, $R_1$ Wasserstoff und $R_2$ 3,5-Dichlorbenzyl, Chinolin-4-yl-methyl, 1-(3-Chlorphenyl)ethyl oder 1-(4-Chlor-3-jod-phenyl)ethyl oder $R_1$ Hydroxy und $R_2$ 3,4-Dimethylbenzyl, 3,4-Methylendioxybenzyl, 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,5-Dimethoxy-phenyl)ethyl, 1-(3,4,5-Trimethoxyphenyl)ethyl,3-Phenylprop-2-yl, 2-(3,4-Dichlorphenyl)propyl, 3-(3,4-Dichlorphenyl)prop-2-yl oder 3-Phenyl-3-hydroxy-prop-2-yl und $R_3$ Wasserstoff bedeutet oder R Cyclohexylmethyl, $R_1$ Wasserstoff, $R_2$ 4-Chlorbenzyl und $R_3$ Methyl bedeutet oder
c) R Cylohex-3-enylmethyl, $R_1$ (S)-Hydroxy, $R_2$ 1(S)-(3,4-Dichlorphenyl)ethyl und $R_3$ Wasserstoff bedeutet oder
d) R Benzyl, $R_1$ Hydroxy, $R_2$ $\alpha$-Cyclopropyl-3,4-dichlor-benzyl, 3,4,5-Trimethoxybenzyl, 1-(3,5-Dimethoxyphenyl)ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,4,5-Trimethoxyphenyl)ethyl, 3-Phenylprop-2-yl, 3-Phenyl-3-hydroxy-prop-2-yl, 1-, 2- oder 3-(3,4-Dichlorphenyl)propyl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet, oder
e) R 4-Chlorbenzyl, 4-Methylbenzyl, 4-Methoxybenzyl oder Cyclohex-3-enylmethyl bedeutet und $R_1$, $R_2$ und $R_3$ Wasserstoff darstellen, und ihre Salze, Verfahren zur Herstellung der erfindungsgemässen Verbindungen, diese enthaltende pharmazeutische Präparate und ihre Verwendung als Arzneimittelwirkstoffe.

Die Verbindungen der Formel I liegen auf Grund ihres amphoteren Charakters in Form innerer Salze vor und können sowohl Säureadditionssalze als auch Salze mit Basen bilden.

Säureadditionssalze von Verbindungen der Formel I sind beispielsweise deren pharmazeutisch verwendbare Salze mit geeigneten Mineralsäuren, wie Halogenwasserstoffsäuren Schwefelsäure oder Phosphorsäure z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Ethyl- oder Diethylamin, Mono-, Di- oder Tri-(hydroxyniederalkyl)aminen, wie Ethanol-, Diethanol- oder Triethanolamin, Tris-(hydroxymethyl)methylamin oder 2-Hydroxytertiärbutylamin, oder N-(Hydroxyniederalkyl)-N,N-diniederalkylaminen bzw. N-(Polyhydroxyniederalkyl)-niederalkylaminen, wie 2-(Dimethylamino)ethanol oder D-Glucamin, oder quaternären aliphatischen Ammoniumhydroxiden, z.B. Tetrabutylammoniumhydroxid.

Abhängig vom Vorhandensein asymmetrischer Kohlenstoffatome können die erfindungsgemässen Verbindungen in Form von Isomerengemischen, speziell als Racemate, oder in Form reiner Isomere, speziell von optischen Antipoden vorliegen.

Die neuen Verbindung der FormelI und ihre pharmazeutisch verwendbaren Salze weisen ebenfalls wertvolle $GABA_B$-antgonistische Eigenschaften auf. Insbesondere zeigen sie eine wirksame Bindung an den $GABA_B$-Rezeptor und erweisen sich als Antagonisten von GABA ($\gamma$-aminobutyric acid) an diesem Rezeptor. Mechanistisch gesehen kann Antagonismus an $GABA_B$-Rezeptoren die Freisetzung von schnellen Reiz-Aminosäuren-Transmittern, d.h. Glutamat

und Aspargat, steigern und so die Informationsverarbeitung im Gehirn verbessern. Damit stimmt die Erkenntnis überein, dass das späte postsynaptische Inhibitionspotential im Hippocampus, das einem $GABA_B$-Mechanismus zugeschrieben wird, durch die Antagonisten abgebaut wird und damit eine schnellere Nervenimpulsübertragungsfolge ermöglicht.

Andererseits wurde gefunden, dass die chronische Behandlung mit Antidepressiva und wiederholte Elektroschocks die Zahl der $GABA_B$-Rezeptoren in der Hirnrinde bei Ratten erhöht. In Übereinstimmung mit Rezeptortheorien sollte die chronische Behandlung mit $GABA_B$-Antagonisten zu derselben Wirkung führen. Aus diesem und anderen Gründen können daher $GABA_B$-Antagonisten als Antidepressiva wirken.

Die erfindungsgemässen neuen $GABA_B$-Antagonisten wechselwirken am $GABA_B$-Rezeptor mit $IC_{50}$-Werten ab etwa $10^{-8}$M (Mol/l) an Rattenhimrindenmembranen. Im Gegensatz zu $GABA_B$-Agonisten wie Baclofen potenzieren sie nicht die Stimulierung von Adenylatcyclase an Rattenhimrindenschnitten durch Noradrenalin, sondern wirken als Antagonist der Baclofen-Wirkung. Der Antagonismus gegenüber Baclofen kann auch an elektrophysiologischen Modellen *in vitro* gezeigt werden, z.B. am Penicillin-induzierten "epileptischen" Hippocampus-Schnittpräparat, wo Baclofen bei einer Konzentration von 6µM (Mikromol/Liter) "epilepsieartige" Entladungen von Pyramidenzellen inhibiert. Die erfindungsgemässen Verbindungen wirken als Antagonist der Baclofen-Wirkung bei Konzentrationen von etwa 10 bis etwa 100µM (Mikromol/Liter). *In vivo* kann der Antagonismus durch lontophorese von Baclofen an Rattenhirnrinde und durch systemische Anwendung von Antagonisten in Dosen von 10-100mg/kg bewiesen werden. Bei Dosen von etwa 30mg/kg tritt Antagonismus gegen die muskelrelaxierende Wirkung von Baclofen ein, die im Rotarod-Modell gemessen wird.

Die neuen $GABA_B$-Antagonisten zeigen nicht nur Antagonismus gegen Baclofen, sondern weisen auch eine eigenständige Wirkung als Antagonisten von endogenem GABA auf. Somit sind die Antagonisten aktiv bei herkömmlichen Verhaltensmodellen, die charakteristisch sind für antidepressive, anxiolytische und/oder nootrope Eigenschaften. Verbindungen der Formel(I), so wurde gefunden, sind bei oraler Anwendung aktiv im Schwimmtest nach Porsolt, im Geller-Test, dem verzögerten passiven Meidungstest (Einversuchs-Modifizierung) in Vorversuchs- und Nachversuchs-Situationen, im Zweikammertest und im komplexen Labyrinth. Ausserdem wurde bei Untersuchungen an Rhesusaffen ein gesteigerter Spieltrieb, Neugierde, soziales Pflegeverhalten und eine Verminderung von Angstmerkmalen beobachtet.

Die neuen Verbindungen der Formel I und ihre pharmazeutisch verwendbaren Salze sind deshalb vorzüglich geeignet als Nootropika, Antidepressiva und Anxiolytika, z.B. zur Behandlung von cerebralen Insuffizienzerscheinungen, Gemütsdepressionen und von Angstzuständen.

Auf Grund des Antagonismus der bereits bekannten $GABA_B$-Antagonisten gegenüber Baclofen wurde bislang davon ausgegangen, dass $GABA_B$-Antagonisten, wie Verbindungen der Formel I, keine antiepileptische Wirkungskomponente besitzen.

Es wurde nun überraschend festgestellt, dass $GABA_B$-Antagonisten, insbesondere Verbindungen der Formel I, *in vivo* ausgeprägte Antiabsenz-Eigenschaften aufweisen.

Diese können an einem bestimmten Rattenstamm anhand ihrer ausgeprägten Hemmwirkung auf spontane "Spike and Wave"-Entladungen in folgendem Tiermodell für Absenz-Epilepsie dokumentiert werden.

Etwa 30% der im Centre de Neurochimie in Strasbourg gezüchteten Wistar-Ratten zeigen spontane Verhaltensveränderungen, unter denen Elektro-Enzephalogramm (EEG) und Symptome den menschlichen Absenzen (Petit mal) vergleichbar sind. Synchrone "Spike and Wave"-Entladungen (SWE; Frontoparietal-Cortex; 7-8 Hz; 300-1000 µV, Dauer 0,5 bis 40 s, Mittelwert = 6,0±3,4 s) und öfters Myoclonia facialis begleiten einen Verhaltensstillstand. Diese Absenzähnliche Zustände treten spontan und wiederholt auf. Durch selektive Züchtung dieser Ratten war es, möglich einen Stamm zu erhalten, bei welchem 100% der Ratten diese SWE zeigen (epileptische Ratten). Umgekehrt konnte ein Stamm gezüchtet werden, bei welchem 100% der Ratten SWE-frei sind (Kontroll-Ratten). Dieses pharmakologische Modell ist beschrieben in Vergnes M., Marescaux C., Micheletti G., Reis J., Depaulis A., Rumbach L. und Warter J.M., Neurosci. Lett. 33, 97-101 (1982).

In diesem Modell vermindern beispielsweise die klinisch verwendeten Antiepileptika Ethosuximid, Diazepam, Trimethadion und Natriumvalproat in Dosen ≥ 25 mg/kg (Ethosuximid,), ≥ 0,5 mg/kg (Diazepam) bzw. ≥ 50 mg/kg (Trimethadion und Natriumvalproat) die Spike and Wave-Entladungen dosisabhängig. Carbamazepin und Phenytoin sind unwirksam oder verschlimmern bei hoher Dosierung die Anfalle. Phenobarbital ist wirksam bei 2,5 bis 10 mg/kg und ist unwirksam bei 20 mg/kg. Die Wirkung dieser Antiepileptika auf die Krisen bei der Ratte und Absenzen beim Menschen unterstützen die Hypothese, dass dieses Tiermodell ein pharmakologisches Modell für Absenz-Epilepsie darstellt. Sein prädiktiver Wert scheint mindestens so gut zu sein wie derjenige anderer gebräuchlicher Tiermodelle.

Die Verbindungen der Formel I und ihre pharmazeutisch verwendbaren Salze sind deshalb neben ihrer Eignung als Nootropika, Antidepressiva und Anxiolytika vorzüglich geeignet als Arzneimittelwirkstoffe in Antiepileptika zur Behandlung von Epilepsien des "petit mal"- Formenkreises, sowohl der spontanen Absenzepilepsien, wie spontanen Absenzepilepsien bei Kindern und Jugendlichen sowie von atypischen Absenzen, wie Absenzen des Lennox-Gastaut-Syndroms, als auch von solchen, die bei der Behandlung mit üblichen "grand mal"-Antiepileptika, wie Phenytoin, Carbamazepin oder Vigabatrin und Antiepileptika mit gleichem oder ähnlichem Wirkungsprofil, als unerwünschte Nebenwirkungen auftreten.

Die Erfindung betrifft beispielsweise Verbindungen der Formel I, worin

a) R Diethoxymethyl, $R_1$ Wasserstoff und $R_2$ 2,6- oder 3,5-Dichlorbenzyl, Pyrid-3-ylmethyl oder 1-(4-Methoxy)phenethyl oder $R_1$ Hydroxy und $R_2$ 3,4-Dichlorbenzyl, 1-(3-Chlor-4-jod-phenyl)ethyl oder 1-(4-Chlor-3-jod-phenyl)ethyl und $R_3$ Wasserstoff bedeutet oder

b) R Cyclohexylmethyl, $R_1$ Wasserstoff und $R_2$ 3,5-Dichlorbenzyl, 1-(3-Chlorphenyl)ethyl oder Chinolin-4-ylmethyl oder $R_1$ Hydroxy und $R_2$ 3,4-Dimethylbenzyl oder 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(3,5-Dimethoxyphenyl)ethyl, 1-(3,4,5-Trimethoxyphenyl)ethyl, 3-Phenylprop-2-yl, 3-(3,4-Dichlorphenyl)prop-2-yl oder 3-Phenyl-3-hydroxy-prop-2-yl und $R_3$ Wasserstoff bedeutet oder $R_1$ Wasserstoff, $R_2$ 4-Chlorbenzyl und $R_3$ Methyl bedeutet oder

c) R Cylohex-3-enylmethyl, $R_1$ (S)-Hydroxy und $R_2$ 1(S)-(3,4-Dichlorphenyl)ethyl und $R_3$ Wasserstoff bedeutet oder

d) R Benzyl, $R_1$ Hydroxy, $R_2$ 3,4,5-Trimethoxybenzyl, 1-(3,5-Dimethoxyphenyl)ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-, 2- oder 3-(3,4-Dichlorphenyl)propyl, 3-Phenylprop-2-yl, 3-Phenyl-3-hydroxy-prop-2-yl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet,

und ihre Salze, insbesondere ihre pharmazeutisch verwendbaren Salze.

Die Erfindung betrifft vor allem Verbindungen der Formel I, worin R Cyclohexylmethyl oder Benzyl bedeutet, $R_1$ Hydroxy ist, $R_2$ für 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,5-Dimethoxy-phenyl)ethyl oder 1-(3,4,5-Trimethoxy)phenethyl steht und $R_3$ Wasserstoff ist, und ihre Salze, insbesondere ihre pharmazeutisch verwendbaren Salze.

Die Erfindung betrifft in allererster Linie Verbindungen der Formel I, worin R Cyclohexylmethyl oder Benzyl, $R_1$ Hydroxy, $R_2$ 1-(3,4,5-Trimethoxyphenyl)ethyl und $R_3$ Wasserstoff ist, und ihre Salze, insbesondere ihre pharmazeutisch verwendbaren Salze.

Die Erfindung betrifft jeweils vorzugsweise diejenigen der vorstehend definierten Verbindungen, in denen $R_1$ Hydroxy bedeutet und das die Hydroxygruppe tragende C-Atom sowie, sofern vorhanden, das α-C-Atom von 1-(3,4-Dichlor-, 3-Chlor-4-jod-, 4-Chlor-3-jod-, 2,4-Dimethoxy-, 2,5-Dimethoxy-, 2,4-Dimethoxy-, 3,4-Dimethoxy-, 3,5-Dimethoxy-, 3,4,5-Trimethoxy bzw. 3,4-Methylendioxyhenyl)ethyl, α-Cyclopropyl-4,4-dichlornenzyl bzw. 1-(3,4-Dichlorphenyl)propyl (S)-Konfiguration aufweisen, wobei R, $R_2$ und $R_3$ die jeweils angegebenen Bedeutungen haben.

Das Verfahren zur Herstellung der erfindungsgemäss bereitgestellten neuen Aminoalkanphosphinsäuren der Formel I ist dadurch gekennzeichnet, dass man in einer Verbindung der Formel II

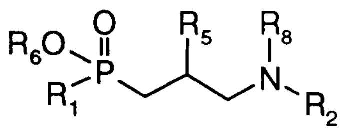

(II),

worin $R_5$ für Wasserstoff oder geschütztes Hydroxy steht, $R_6$ für eine Hydroxyschutzgruppe, $R_8$ für eine Gruppe $R_3$ oder für eine Aminoschutzgruppe und R und $R_2$ die angegebenen Bedeutungen haben, oder in einem Salz davon die Hydroxygruppen durch Ersatz der Hydroxyschutzgruppe $R_6$ durch Wasserstoff freisetzt und gegebenenfalls die Aminoschutzgruppe $R_8$ abspaltet und gegebenenfalls die Hydroxygruppen $R_1$ aus der geschützten Hydroxygruppe $R_5$ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

Die Ausgangsstoffe der Formel II können auf verschiedenen Wegen hergestellt werden, beispielsweise indem man

a) in eine Verbindung der Formel III

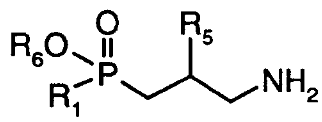

(III),

worin R die angegebene Bedeutung hat, $R_5$ Wasserstoff oder geschütztes Hydroxy darstellt und $R_6$ für eine Hydroxyschutzgruppe steht, die Gruppe $R_2$ und gewünschtenfalls Methyl $R_3$ einführt oder

b) eine Verbindung der Formel IV

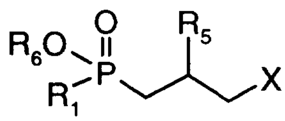

(IV),

worin R die angegebene Bedeutung hat, $R_5$ Wasserstoff oder geschütztes Hydroxy darstellt, $R_6$ für eine Hydroxyschutzgruppe steht und X eine reaktionsfähige veresterte Hydroxygruppen bedeutet, oder ein Salz davon mit einer Verbindung der Formel V

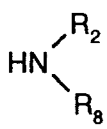

(V),

worin $R_2$ die angegebene Bedeutung hat und $R_8$ eine Gruppe $R_3$ oder eine Aminoschutzgruppe bedeutet, umsetzt oder

c) eine Verbindung der Formel VI

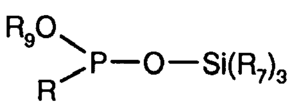

(VI),

worin $R_9$ für eine Hydroxyschutzgruppe $R_6$ oder $-Si(R_7)_3$ steht und R die angegebene Bedeutung hat, wobei die Reste $R_7$ gleiche oder verschiedene aliphatische Kohlenwasserstoffreste, beispielsweise Niederalkyl, insbesondere Methyl und/oder Tertiärbutyl, bedeuten, mit einer Verbindung der Formel VII

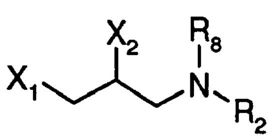

(VII),

worin $X_1$ reaktionsfähige verestertes Hydroxy und $X_2$ Wasserstoff bedeutet oder $X_1$ und $X_2$ gemeinsam für Epoxy stehen und $R_8$ eine Gruppe $R_3$ oder eine Aminoschutzgruppe bedeutet, kondensiert oder

d) eine Verbindung der Formel VIII

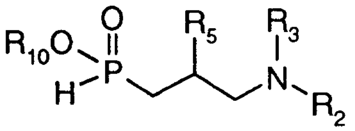

(VIII),

worin $R_5$ für Wasserstoff oder geschütztes Hydroxy steht, $R_{10}$ Wasserstoff oder eine Gruppe $R_6$ darstellt und $R_2$ und $R_3$ die angegebenen Bedeutungen haben mit einem Silylierungsmittel umsetzt und die erhaltene silylaktivierte Verbindung der Formel IX

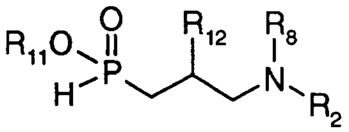

(IX),

in der $R_8$ für eine von Wasserstoff verschiedene Gruppe $R_3$ oder eine Gruppe der Formel $-Si(R_7)_3$ steht, $R_{11}$ eine Gruppe $R_6$ oder eine Gruppe $-Si(R_7)_3$ bedeutet und $R_{12}$ Wasserstoff, eine Gruppe $R_5$ oder eine Gruppe der Formel $-OSi(R_7)_3$ darstellt, wobei die Reste $R_7$ gleiche oder verschiedene aliphatische Kohlenwasserstoffreste, beispielsweise Niederalkyl, insbesondere Methyl und/oder Tertiärbutyl, bedeuten, mit einem reaktionsfähigen Ester eines aliphatischen, cycloaliphatischen, cyloaliphatisch-aliphatischen oder araliphatischen Alkohols, mit einem in $\alpha,\beta$-Stellung eine gegebenenfalls zusätzlich Doppelbindung aufweisenden aliphatischen, cycloaliphatischen, cyloaliphatisch-aliphatischen, araliphatischen oder heteroarylaliphatischen Kohlenwasserstoff, mit einem aliphatischen, cycloaliphatischen, cyloaliphatisch-aliphatischen, araliphatischen oder heteroarylaliphatischen Aldehyd oder Keton oder mit einem aliphatischen Epoxid umsetzt oder

e) zur Herstellung einer Verbindung der Formel II, in der $R_2$ Hydroxy bedeutet, eine Verbindung der Formel X

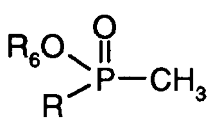

(X)

in der Form eines Metallsalzes der Formel XI

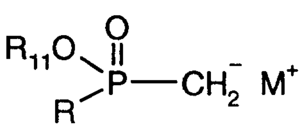

(XI),

worin $R_{11}$ eine Gruppe $R_6$ oder $-Si(R_7)_3$ und R die angegebene Bedeutung hat, in dem die Reste $R_7$ gleiche oder verschiedene aliphatische Kohlenwasserstoffreste, beispielsweise Niederalkyl, insbesondere Methyl und/oder Tertiärbutyl, bedeuten, und $M^+$ ein Alkali-, Erdalkali- oder Übergangsmetall-Kation darstellt, mit einem Aldehyd der Formel XII

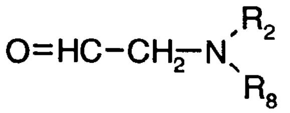

(XII)

umsetzt worin $R_2$ die angegebene Bedeutung hat und $R_8$ eine Gruppe $R_3$ oder eine Aminoschutzgruppe bedeutet.

Gewünschtenfalls kann man jeweils in eine primär erhaltene Verbindung der Formel II, worin $R_3$ Wasserstoff darstellt, Methyl $R_3$ einführen.

Bei den erfindungsgemässen pharmazeutischen Präparaten handelt es sich jeweils solche in einer Dosiseinheitsform, welche eine therapeutisch wirksame Menge der Aktivsubstanz, allein oder zusammen mit einem pharmazeutisch anwendbaren Träger, insbesondere mit anorganischen oder organischen, festen oder flüssigen, pharmazeutisch verwendbaren Trägerstoffen enthalten, sodass sie sich zur enteralen, wie oralen, ferner rektalen, und parenteralen Verabreichung an Warmblüter eigen.

Die erfindungsgemäss bereitgestellten pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Injektions- oder Infusionslösungen, vorzugsweise in Ampullen. Diese Formulierungen werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemische oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyethylenglykol, gelöst oder suspendiert, wobei ebenfalls Stabilisatoren zugefügt sein können.

Als rektal anwendbare pharmazeutische Präparate kommen z. B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyethylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die pharmazeutischen Präparate können sterilisiert sein und, wenn erwünscht, weitere pharmakologisch wirksame Stoffe und/oder Hilfsstoffe, z.B. Konservierungs-, Stabilisier-, Netz- und/oder Emulgiermittel, Löslichkeitsvermittler,

Salze zur Regulierung des osmotischen Druckes und/oder Puffer enthalten.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 und etwa 60 mg/kg, insbesondere 10 und etwa 40 mg/kg und für Warmblüter mit einem Körpergewicht von etwa 40 kg vorzugsweise zwischen etwa 200 mg und etwa 2400 mg, insbesondere etwa 400 bis etwa 1600 mg, die zweckmässigerweise auf 2 bis 6, z.B. 3 oder 4 Einzeldosen aufgeteilt wird.

Die nachfolgenden Beispiele dienen zur Illustration der Erfindung; Temperaturen sind in Celsiusgraden, Drucke in mbar angegeben.

Beispiel 1: Zu einer Lösung von 1.61 g 3-(3,5-Dichlorbenzylamino)propyl(diethoxymethyl)phosphinsäureethylester in 3 ml Ethanol fügt man eine Lösung von 0,36 g Lithiumhydroxid-Monohydrat in 7 ml Wasser hinzu und erwärmt 24 Stunden auf 60°. Dann kühlt man auf Raumtemperatur und zieht unter vermindertem Druck das Lösungsmittel ab. Der Eindampfrückstand wird in Wasser aufgenommen und mit Phosphorsäure neutral gestellt. Es bildet sich eine weisser Niederschlag. Dieser wird abfiltriert und das Filtrat zur Trockne eingedampft. Der weisse Eindampfrückstand wird unter vermindertem Druck getrocknet und aus Toluol/Diethylether kristallisiert. Nach Absaugen und Trocknen erhält man 3-(3,5-Dichlorbenzylamino)propyl(diethoxymethyl)phosphinsäure vom Smp. 160-161°.

Das Ausgangsmaterial kann z.B. folgendermassen hergestellt werden:

Eine Lösung von 2,53 g 3-Aminopropyl(diethoxymethyl)phosphinsäureethylester in 10 ml wasserfreiem Methanol wird mit 1,41 g 3,5-Dichlorbenzaldehyd versetzt und die erhaltene klare Lösung 30 Minuten bei Raumtemperatur gerührt. Dann fügt man zunächst 0,6 g Eisessig und dann tropfenweise 0,21 g Natriumcyanoborhydrid, gelöst in 5 ml Methanol hinzu. Es setzt eine exotherme Reaktion ein. Man rührt 3 Stunden bei 20°, stellt auf pH 8 ein und zieht das Lösungsmittel ab. Der Rückstand wird in Dichlormethan gelöst und mit Wasser gewaschen. Die organische Phase wird abgetrennt, über Natriumsulfat getrocknet und zur Trockne eingedampft. Das zurückbleibende Öl wird an Kieselgel chromatographisch gereinigt. Man erhält 3-(3,5-Dichlorbenzylamino)propyl(diethoxymethyl)phosphinsäureethylester als fahlgelbes Öl.

Beispiel 2: Eine Lösung von 4.1 g 3-{N-1-(3,4-Dichlorphenyl)ethyl]amino}-2(S)-hydroxy-propyl(cyclohexylmethyl)phosphinsäureethylester in 30 ml 5n-Salzsäure wird 24 Stunden zum Rückfluss erhitzt, wobei sich 2 Phasen bilden. Man kühlt auf Raumtemperatur, zieht das Lösungsmittel unter vermindertem Druck ab, nimmt in absolutem Ethanol auf und destilliert das Restwasser azeotrop ab. Der zurückbleibende weisse Feststoff wird aus Isopropanol kristallisiert und ergibt 3-{N-1-(3,4-Dichlorphenyl)ethyl]amino}-2(S)-hydroxy-propyl(cyclohexylmethyl)phosphinsäure-hydrochlorid vom Smp. 174-180°.

Das Ausgangsmaterial kann wie folgt hergestellt werden:

Eine Suspension von 26.4 g 99 %-igem Natriumhydrid in 1500 ml Tetrahydrofuran wird unter Argon innerhalb von 3 Stunden tropfenweise mit einer Lösung von 221 g Diethoxymethylphosphinsäureethylester in 1000 ml Tetrahydrofuran versetzt, wobei die Temperatur bei 20°-25° gehalten wird. Die Reaktion ist exotherm und mit Gasentwicklung verbunden. Man lässt 2 Stunden bei Raumtemperatur nachrühren und fügt dann innerhalb von 20 Minuten 177.1 g Brommethylcyclohexan hinzu und lässt 24 Stunden am Rückfluss nachrühren, kühlt auf 0° und gibt vorsichtig 200 ml Wasser hinzu. Das Lösungsmittel wird unter vermindertem Druck abgezogen und der Rückstand zwischen Dichlormethan und Wasser verteilt. Die organische Phase wird abgetrennt, über Natriumsulfat getrocknet und unter vermindertem Druck eingedampft. Das zurückbleibende Öl wird unter vermindertem Druck destilliert und ergibt Diethoxymethyl(cyclohexylmethyl)phosphinsäureethylester vom Kp = 85° ($6x10^{-4}$ bar).

Eine Lösung von 151 g Diethoxymethyl(cyclohexylmethyl)phosphinsäureethylester in 430 ml Dichlormethan, enthaltend 10 Vol.-% Ethanol wird mit 107 g Trimethylchlorsilan versetzt und 3 Tage bei Raumtemperatur stehengelassen, dann zur Trockne eingedampft und unter vermindertem Druck destilliert. Man erhält Cyclohexylmethylphosphinsäureethylester vom Kp = 50° ($3x10^{-4}$ bar).

20.0 g Cyclohexylmethylphosphinsäureethylester werden in 150 ml Tetrahydrofuran gelöst, bei 0° mit 11,0 g Triethylamin und dann tropfenweise mit 12.0 g Trimethylchlorsilan versetzt, wobei sich ein weisser Niederschlag bildet. Die erhaltene Suspension wird 24 Stunden bei Raumtemperatur gerührt und dann unter Argon filtriert. Das Filtrat wird unter vermindertem Druck eingedampft. Der Rückstand wird mit 10.9 g (R)-Epichlorhydrin und 2,0 g wasserfreiem Zinkchlorid versetzt. Nach Abklingen der exothermen Reaktion wird weitere 24 Stunden bei 60° zum Rückfluss erhitzt. Man lässt auf Raumtemperatur abkühlen, verdünnt mit Dichlormethan und wäscht mit Wasser. Die organische Phase wird abgetrennt, über Nariumsulfat getrocknet und eingedampft. Der Rückstand wird in Dichlormethan aufgenommen und mit Wasser gewaschen. Trocknen über Natriumsulfat und Eindampfen ergibt ein Öl. Dieses wird mit 1%-iger methanolischer Essigsäure aufgenommen, 24 Stunden bei Raumtemperatur stehengelassen und eingedampft. Der Rückstand wird an Silicagel chromatographisch gereinigt. Man erhält 3-Chlor-2(R)-hydroxy-propyl-(cyclohexylmethyl)-phosphinsäureethylester; $^1H$-NMR-Spektrum (in $CDCl_3$): $\delta$ = 4.47 (1H, d), 4.21-4.02 (3H, m), 3.6 (2H, m), 2.11-1.54 (9H, m), 1.38-0.97 (9H, m).

Eine Mischung von 4.24 g 3-Chlor-2(R)-hydroxy-propyl(cyclohexylmethyl)phosphinsäureethylester, 2.85 g 1-(3,4-Dichlorphenyl)ethylamin, 1.95 g Hünig'scher Base und 15 ml Ethanol werden 4 Tage zum Rückfluss erhitzt.

Nach Abkühlen auf Raumtemperatur und Abziehen des Lösungsmittels unter vermindertem Druck verteilt man zwischen Dichlormethan und Wasser, trocknet über Natriumsulfat und dampft erneut zur Trockne ein. Chromatographie an Silicagel ergibt 3-{N-1-(3,4-Dichlorphenyl)ethyl]amino}-2(S)-hydroxy-propyl-(cyclohexylmethyl)phosphinsäureethylester $[\alpha]_{20}^{D}$ = -1.8 ± 0.9° (c= 1.079 in Chloroform).

Beispiel 3: In analoger Weise wie in Beispiel 1 beschrieben kann man 3-[N-(2,6-Dichlorbenzyl)amino]propyl-(diethoxymethyl)-phosphinsäure, Smp. 171-173°, herstellen.

Beispiel 4: In analoger Weise wie in Beispiel 1 beschrieben kann man 3-[N-(Pyrid-3-ylmethylamino)propyl-(diethoxymethyl)-phosphinsäure, Smp. 169-171°, herstellen.

Beispiel 5: In analoger Weise wie in Beispiel 1 beschrieben kann man 3-{N-[1-(4-Methoxyphenyl)ethylamino}propyl-(diethoxymethyl)-phosphinsäure, Smp. 144-146°, herstellen.

Beispiel 6: Eine Lösung von 1.1 g 3-{N-1(S)-(3,4-Dichlorphenyl)ethyl]amino}-2(S)-hydroxy-propyl-(cyclohex-3-enylmethyl)-phosphinsäureethylester (erhältlich wie in Beispiel 2 beschrieben ausgehend von Brommethylcyclohex-3-en und 1,1-Diethoxyethylphosphinsäureethylester über 3-Chlor-2(R)-hydroxy-propyl(cyclohex-3-enylmethyl)-phosphinsäureethylester und Weiterumsetzung desselben mit 1(S)-(3,4-Dichlorphenyl)ethylamin) in 5 ml Ethanol wird mit einer Lösung von 0.13 g Lithiumhydroxid in 3 ml Wasser versetzt, 24 Stunden auf 60° erwärmt, dann auf Raumtemperatur abgekühlt und unter vermindertem Druck eingedampft. Der Rückstand wirdin Wasser aufgenommen, mit wässriger Phosphorsäurelösung neutralgestellt und erneut unter vermindertem Druck eingedampft. Der zurückbleibende weisse Festkörper wird in heissem Methanol aufgenommen und filtriert. Nach Abziehen des Methanols erhält man einen hygroskopischen Feststoff der nach Kristallisation aus Cyclohexan/Petrolether (60-80°) 3-{N-1(S)-(3,4-Dichlorphenyl)ethyl]amino]-2(S)-hydroxy-propyl(c yclohex-3-enylmethyl)-phosphinsäure als hygroskopischen Feststoff ergibt; $^1$H-NMR-Spektrum (in $CD_3OD$): δ = 7.68 (1H), 7.58 (1H), 7.40 (1H), 6.60 (2H, CH=CH), 4.22 (1H, q, CH), 4.13 (1H, m), 2.97 (1H, dd, CHN), 2.63 (1H, dd, CHN), 2.33-1,21 (14H, m). Das Hydrochlorid schmilzt bei 205-206°.

Beispiel 7: In analoger Weise wie in Beispiel 2 beschrieben kann man 3-[N-(3,4,5-Trimethoxybenzyl)amino]-2(S)-hydroxy-propyl-(benzyl)-phosphinsäure, Smp. 100-102°, herstellen.

Beispiel 8: In analoger Weise wie in Beispiel 2 beschrieben kann man

3-{N-[1-(3,4-Dichlorphenyl)ethyl]amino}-2(S)-hydroxy-propyl-(benzyl)-phosphinsäure, Smp. 150-158°;
3-[N-(3,4-Dichlor-αcyclopropyl-benzyl)amino]-2(S)-hydroxy-propyl-(benzyl)-phosphinsäure, Smp. 204-207°;
3-{N-[1(S)-(3,4-Dichlorphenyl)ethyl]amino}-2(R)-hydroxy-propyl-(benzyl)-phosphinsäure, Smp. 188-190°, und
3-{N-[1(R)-(3,4-Dichlorphenyl)ethylamino]-2(S)-hydroxy-propyl-(benzyl)-phosphinsäure, $^1$H-NMR-Spektrum (in $CD_3OD$): δ = 7.73 (1H, m, Phenyl), 7.62 (1H, m, Phenyl), 7.45 (1H, m, Phenyl), 7.37-7.17 (5H, m, Phenyl), 4.41 (1H, q, CHN), 4.21 (1H, q, CHN), 3.18 (2H, d, P-$CH_2$-Phenyl), 3.05-2.88 (2H, m, $CH_2N$), 1.95 (2H, m, $CH_2$-P), 1.65 (3H, d, $CH_3$), herstellen.

Beispiel 9: In analoger Weise wie in Beispiel 2 beschrieben kann man 3-[N-(3,4-Dimethylbenzyl)amino]-2(S)-hydroxy-propyl-(cyclohexylmethyl)-phosphinsäure, Smp. 164-166° herstellen.

Beispiel 10: In analoger Weise wie in Beispiel 2 beschrieben kann man 3-{N-[1-(4-Chlor-3-jod-phenyl)ethyl]amino}-2(S)-hydroxy-propyl-(benzyl)-phosphinsäurehydrochlorid, Smp. 118-124° herstellen.

Beispiel 11: In analoger Weise wie in Beispiel 6 beschrieben kann man 3-{N-[1-(3,4,5-Trimethoxyphenyl)ethylamino)-2(S)-hydroxy-propyl-(cyclohexylmethyl)-phosphinsäure-hydrochlorid, Smp. 175-182°, sowie 3-{N-[1(S)-(3,4,5-Trimethoxyphenyl)ethylamino)-2(S)-hydroxy-propyl-(cyclohexylmethyl)-phosphinsäure, Smp. 208-209°, herstellen.

Beispiel 12: In analoger Weise wie in Beispiel 6 beschrieben kann man

3-{N-[1-(3,5-Dimethoxyphenyl)ethylamino)-2(S)-hydroxy-propyl-(benzyl)-phosphinsäure, Smp. 150-165°;
3-{N-[1-(2,5-Dimethoxyphenyl)ethylamino)-2(S)-hydroxy-propyl-(benzyl)-phosphinsäure, Smp. 182-191°;
3-{N-[1-(2,6-Dimethoxyphenyl)ethylamino)-2(S)-hydroxy-propyl-(benzyl)-phosphinsäure, Smp. 130-162°;
3-{N-[1-(3,4-Methylendioxyphenyl)ethyl]amino}-2(S)-hydroxy-propyl-(benzyl)-phosphinsäure, Smp. 200-219°;
3-{N-[1-(3,4-Dimethoxyphenyl)ethylamino)-2(S)-hydroxy-propyl-(benzyl)-phosphinsäure, Smp. 199-208°, und
3-{N-[1-(2,4-Dimethoxyphenyl)ethylamino)-2(S)-hydroxy-propyl-(benzyl)-phosphinsäure, Smp. 192-202°, herstellen.

Beispiel 13: In analoger Weise wie in Beispiel 6 beschrieben kann man

3-{N-[1-(2,5-Dimethoxyphenyl)ethylamino}-2(S)-hydroxy-propyl-(cyclohexylmethyl)-phosphinsäure` Smp. 140-149°;
3-{N-[1-(2,6-Dimethoxyphenyl)ethylamino}-2(S)-hydroxy-propyl-(cyclohexylmethyl)-phosphinsäure, Smp. 172-185°;

3-{N-[1-(3,4-Dimethoxyphenyl)ethylamino}-2(S)-hydroxy-propyl-(cyclohexylmethyl)-phosphinsäure, Smp. 189-195°;

3-{N-[1-(2,4-Dimethoxyphenyl)ethylamino}-2(S)-hydroxy-propyl-(cyclohexylmethyl)-phosphinsäure, Smp. 156-171°;

3-{N-[1-(3,4-Methylendioxyphenyl)ethylamino}-2(S)-hydroxy-propyl-(cyclohexylmethyl)-phosphinsäure, Smp. 190-202°;

3-[N-(3,4-Methylendioxybenzyl)amino]2(S)-hydroxy-propyl-(cyclohexylmethyl)-phosphinsäure, Smp. 176-178°, und

3-{N-[1-(3,5-Dimethoxyphenyl)ethylamino)-2(S)-hydroxy-propyl-(cyclohexylmethyl)-phosphinsäure, $^1$H-NMR ($CD_3OD$): δ= 6.67 (3H, m), 6.53 (1H, m), 4.38-4.11 (2H, m), 3.85 (6H, s), 3.2-2.69 (2H, m), 2.10-1.58 (12H, m), 1.40-0.95 (4H, m), herstellen.

Beispiel 14: Eine Lösung von 0.125 g 3-{N-[1-(3,4-Dichlorphenyl)ethyl]amino}-2(S)-hydroxy-propyl-(cyclohex-3-enylmethyl)-phosphinsäure in 5 ml Ethanol wird mit 20 mg 5%-iger Palladiumkohle versetzt und bei Raumtemperatur und Normaldruck 15 Minuten hydriert. Der Katalysator wird durch Celite abfiltriert und des Filtrat mit ethanolischer Salzsäure auf pH 1 gestellt. Abziehen des Lösungsmittels und umkristallisieren aus Isopropanol ergibt 3-{N-[1-(3,4-Dichlorphenyl)ethyl]amino}-2(S)-hydroxy-propyl-(cyclohexylmethyl)-phosphinsäure, Smp. 173-178°.

Beispiel 15: Eine Lösung von 6.27 g 3-Aminopropyl-(cyclohexylmethyl)-phosphinsäure, und 5.0 g 3,5-Dichlorbenzaldehyd und 1.91 g Eisessig in 50 ml wasserfreiem Methanol wird portionsweise mit 2.0 g Natriumcyanoborhydrid versetzt und 4 Tage bei Raumtemperatur gerührt. Das Lösungsmittel wird abgezogen, der weisse Rückstand in 2.0-m Salzsäure aufgenommen und abfiltriert. Das Filtrat wird zur Trockne eingedampft und der zurückbleibende weisse Feststoff in Essigsäureethylester suspendiert, abfiltriert und aus Propanol kristallisiert. Man erhält 3-[(3,5-Dichlorbenzyl)amino]-propyl-(cyclohexylmethyl)-phosphinsäure, Smp. 231-233°.

Beispiel 16: In analoger Weise wie in Beispiel 15 beschrieben kann man 3-[N-(Chinolin-4-ylmethylamino)-propyl-(cyclohexylmethyl)-phosphinsäure, Smp. 175-177° (Zers.) herstellen.

Beispiel 17: In analoger Weise wie in Beispiel 15 beschrieben kann man 3-[N-(4-Chlorbenzyl)-N-methyl-amino]-propyl-(cyclohexylmethyl)-phosphinsäure, Smp. 211-213°, herstellen.

Beispiel 18: In analoger Weise wie in Beispiel 15 beschrieben kann man 3-[N-(3,4-Chlorbenzyl)amino]-propyl-(butyl)-phosphinsäure, Smp. 216-217° herstellen.

Beispiel 19: In analoger Weise wie in Beispiel 15 beschrieben kann man 3-{N-[1-(4-Chlorphenyl)ethylamino]}-propyl-(butyl)-phosphinsäure, Smp. 177-179° herstellen.

Beispiel 20: In analoger Weise wie in Beispiel 15 beschrieben kann man 3-{N-[1-(3,4-Dichlorphenyl)ethylamino]}-propyl-(butyl)-phosphinsäure, Smp. 134-136° herstellen.

Beispiel 21: In analoger Weise wie in Beispiel 2 beschrieben kann man 3-{N-[1-(3-Chlor-4-jod-phenyl)ethyl]amino}-2(S)-hydroxy-propyl-(cyclohexylmethyl)-phosphinsäure, Smp. 120-155°, herstellen.

Das Ausgangsmaterial kann folgendermassen hergestellt werden:

Eine Lösung von 1.86 g 3-Chlor-4-jod-acetophenon und 5.0 g Ammoniumacetat in 25 ml Methanol werden mit 0.41 g Natriumcyanoborhydrid versetzt und 20 Stunden bei Raumtemperatur gerührt. Man kühlt auf 4° ab, stellt mit Salzsäure auf pH 1, dampft unter vermindertem Druck ein, nimmt den Rückstand in Wasser auf und wäscht mit Ether. Die wässrige Phase wird mit Kaliumhydroxid auf pH 10 gestellt und mit Ether extrahiert. Der Etherextrakt wird über Natriumsulfat getrocknet und eingedampft. Das als Öl zurückbleibende freie Amin wird in Ether gelöst und mit ethanolischer Salzsäure auf pH 1 gestellt. Es bildet sich ein weisser Rückstand, der abfiltriert und unter vermindertem Druck getrocknet wird. Man erhält 3-Chlor-4-jod-benzylammoniumchlorid vom Smp. 220-222°.

Beispiel 22: In analoger Weise wie in Beispiel 21 beschrieben kann man 3-{N-[1-(4-Chlor-3-jod-phenyl)ethyl]amino}-2(S)-hydroxy-propyl-(cyclohexylmethyl)-phosphinsäure, Smp. 118-124° herstellen.

Beispiel 23: In analoger Weise wie in Beispiel 6 beschrieben kann man 3-{N-[1-(4-Chlor-3-jod-phenyl)ethylamino]propyl-(diethoxymethyl)-phosphinsäure herstellen.

Beispiel 24: In analoger Weise wie in Beispiel 2 beschrieben kann man 3-{N-[1(S)-(3,4-Dichlorphenyl)ethyl]amino}-2(S)-hydroxy-propyl-(cyclohexylmethyl)-phosphinsäure-hydrochlorid, Smp. 207.5-209° herstellen.

Beispiel 25: In analoger Weise wie in Beispiel 2 beschrieben kann man 3-{N-[1(S)-(3,4-Dichlorphenyl)ethyl]amino}-2(R)-hydroxy-propyl-(cyclohexylmet hyl)-phosphinsäure-hydrochlorid herstellen; $^1$H-NMR ($CD_3OD$): δ= 7.71 (1H), 7.63 (1H), 7.44 (1H), 4.45 (1H, q), 4.20 (1H), 3.02 (2H, $CH_2N$), 2.10-1.59 (12H, m), 1.39-0.95 (6H, m).

Das Ausgangsmaterial, 1(S)-(3,4-Dichlorphenyl)ethylamin, kann z.B. folgendermassen hergestellt werden:

Eine Lösung von 22.0 g 1-(3,4-Dichlorphenyl)ethylamin in 58 ml 60° warmem Ethanol wird mit einer 60° warmen Lösung von 10.65 g L-(+)-Mandelsäure in 39 ml Ethanol versetzt. Man lässt langsam auf Raumtemperatur abkühlen, filtriert nach 48 bis 72 Stunden den Feststoffanteil ab, wäscht mit kaltem Ethanol und trocknet unter vermindertem Druck bei 60°. Umkristallisation aus Ethanol ergibt das optisch reine Diasteremerensalz. Behandlung desselben mit Natriumhydroxid und anschliessende Extraktion mit Ether ergibt nach Abziehen des Ethers (-)-[1(S)-(3,4-Dichlorphenyl)ethyl]amin, $[\alpha]^D_{20}$ = -26.3 ± 1.0° (c= 0.995 in Methanol).

Die Mutterlaugen werden zur Trockne eingedampft, der erhaltene Rückstand mit wässriger Natronlauge behandelt und mit Ether extrahiert. Das Zurückbleibende Öl (12 g) wird wie vorstehend beschrieben mit 10.5 g D-(-)-Mandelsäure behandelt und ergibt nach Umkristallisieren und Aminfreisetzung (+)[1(R)-(3,4-Dichlorphenyl)ethyl]amin $[\alpha]^D_{20}$ = +25 ± 1.0° (c= 0.995 in Methanol).

Beispiel 26: In analoger Weise wie in Beispiel 2 beschrieben kann man 3-{N-[1(R)-(3,4-Dichlorphenyl)ethyl]amino}-2(R)-hydroxy-propyl-(cyclohexylmethyl)-phosphinsäure-hydrochlorid herstellen.

Das Ausgangsmaterial, 1(R)-(3,4-Dichlorphenyl)ethylamin, kann z.B. folgendermassen hergestellt werden:

Die Mutterlaugen der Racemattrennung gemäss Beispiel 21 werden zur Trockne eingedampft, der erhaltene Rückstand mit wässriger Natronlauge behandelt und mit Ether extrahiert. Das zurückbleibende Öl (12 g) wird wie vorstehend beschrieben mit 10.5 g D-(-)-Mandelsäure behandelt und ergibt nach Umkristallisieren und Aminfreisetzung (+)[1(R)-(3,4-Dichlorphenyl)ethyl]amin, $[\alpha]^D_{20}$ = +25 ± 1.0° (c= 0.995 in Methanol).

Beispiel 27: In analoger Weise wie in Beispiel 2 beschrieben kann man 3-{N-[1(R)-(3,4-Dichlorphenyl)ethyl]amino}-2(S)-hydroxy-propyl-(cyclohexylmethyl)-phosphinsäure-hydrochlorid, Smp. 184-186° herstellen.

Beispiel 28: In analoger Weise wie in Beispiel 21 beschrieben kann man 3-{N-[1-(3-Chlor-4-jod-phenyl)ethyl]amino}-2(S)-hydroxy-propyl-(cyclohexylmethyl)-phosphinsäure-hydrochlorid herstellen; $^1$H-NMR ($CD_3OD$): δ= 8.04 (1H, d), 7.71 (1H, d), 7,20(1H, dd), 4.45 (1H, q), 4.23 (1H, m), 3.25-3.07 (1H, dd), 3.05-2.80 (1H, dd), 2.10-1.58 (12H, m), 1.56-0.99 (6H, d).

Beispiel 29: In analoger Weise wie in Beispiel 21 beschrieben kann man 3-{N-[1-(3-Chlor-4-jod-phenyl)ethyl]amino}-2(S)-hydroxy-propyl-(benzyl)-phosphinsäure-hydrochlorid, Smp. 198-210° herstellen.

Beispiel 30: In analoger Weise wie in Beispiel 21 beschrieben kann man 3-{N-[1-(3-Chlor-4-jod-phenyl)ethylamino)propyl-(diethoxymethyl)-phosphinsäure und 3-{N-[1-(3-Chlor-4-jod-phenyl)ethyl]amino}propyl-(cyclohexylmethyl)-phosphinsäure, Smp. 184-186°, herstellen.

Beispiel 31: Eine Lösung von 2.05 g 3-{N-[1(S)-(3,4,5-Trimethoxyphenyl)-ethyl]amino}-2(S)-hydroxy-propyl-(benzyl)-phosphinsäureethylester in 4 ml Wasser wird mit 10 ml Wasser und 0.24 g Lithiumhydroxid versetzt und über Nacht zum Rückfluss erhitzt. Die nunmehr klare Lösung wird auf Raumtemperatur abgekühlt, mit Phosphorsäure auf pH 7 gestellt und unter vermindertem Druck zur Trockne eingedampft. Der Eindampfrückstand wird in heissem Methanol aufgeschlämmt und filtriert. Nach Eindampfen des Filtrates hinterbleibt ein Schaum, der nach Kristallisation aus Isopropanol 3-{N-[1(S)-(3,4,5-Trimethoxyphenyl)ethyl]amino}-2(S)-hydroxy-propyl-(benzyl)-phosphinsäure vom Smp. 204-206°, $[\alpha]^D_{20}$ = -35.2 ± 1.2° (c= 0.812 in Methanol).

Das Ausgangsmaterial kann z.B. folgendermassen hergestellt werden:

Durch Kristallisation des Mandelsäuresalzes von 1-(3,4,5-Trimethoxyphenyl)-ethylamin aus Ethanol und Freisetzung der freien Base ergibt enantiomerenreines 1(S)-(3,4,5-Trimethoxyphenyl)ethylamin, welches in analoger Weise wie in Beispiel 2 beschrieben mit 3-Chlor-2(R)-hydroxy-propyl-(benzyl)phosphinsäureethylester umgesetzt werden kann.

Beispiel 32: Eine Lösung von 2.26 g 3-[N-(3,4-Dichlorbenzyl)amino]-2(S)-hydroxypropyl(diethoxymethyl)phosphinsäureethylester in 3 ml Ethanol wird mit 0.254 g Lithiumhydroxid und 6 ml Wasser versetzt und 24 Stunden bei 60° gerührt. Man lässt auf Raumtemperatur abkühlen, stellt mit wässriger Phosphorsäure auf pH 7 ein und dampft zur Trockne ein. Der Eindampfrückstand wird in heissem Methanol aufgeschlämmt und filtriert. Das Filtrat wird zur Trockne eingedampft und der glasartige Eindampfrückstand in wenig Ethanol aufgenommen. Man stellt mit ethanolischer Salzsäure auf pH 1 ein, zieht das Lösungsmittel ab und kristallisiert aus Acetonitril man erhält 3-[N-(3,4-Dichlorbenzyl)amino]-2(S)-hydroxy-propyl(diethoxymethyl)phosphinsäure; $[\alpha]^D_{20}$ = -10.8 ± 1.7° (c = 0.595 in Methanol); $^1$H-NMR ($CD_3OD$): δ= 7.76 (1H, m), 7.61 (1H, m), 7.45 (1H, m), 4.47 (1H, d), 4.29 (1H, m), 4.20 (2H, m), 3.80 (2H, m), 3.69 (2H, m), 3.25 (1H, dd), 3.02 (1H, d, d), 2.04 (1H, dd), 1.87 (1H, d, d); 1.20 (6H, t).

Das Ausgangsmaterial kann z. B. folgendermassen hergestellt werden:

Eine Lösung von 227 g Diethoxymethylphosphinsäureethylester in 1500 ml Diethylether und 145 ml Triethylamin wird unter Rühren unter einer Argonatmosphäre tropfenweise mit 133 ml Trimethylchlorsilan versetzt (1 Stunde). Unter leicht exothermer Reaktion bildet sich ein weisser Niederschlag. Man lässt 20 Stunden bei Raumtemperatur nachrühren, filtriert unter Argon und dampft zu Trockne ein. Das zurückbleibende Öl wird mit 63 g (R)-Epichlorhydrin versetzt. Nach Abklingen der exothermen Reaktion erwärmt man 24 Stunden auf 70°. lässt dann auf Raumtemperatur abkühlen, verdünnt mit Dichlormethan und wäscht mit Wasser. Die organische Phase wird abgetrennt, über Natriumsulfat getrocknet und eingedampft. Der Eindampfrückstand wird in 220 ml 1 %-iger methanolischer Essigsäure aufgenommen, 24 Stunden bei Raumtemperatur gerührt und erneut zur Trockne eingedampft. Der Eindampfrückstand wird an Silicagel chromatographisch gereinigt und ergibt 3-Chlor-2(R)hydroxy-propyl-(diethoxymethyl)phosphinsäureethylester als farbloses Öl; $[\alpha]^D_{20}$ = -16.0 ± 1.2° (c = 0.86 in Chloroform); $^1$H-NMR ($CD_3Cl_3$): δ= 4.70 (1Hd, CHP), 4.34 (1H, m, CHO), 4.20 (1H, m, $CH_2OP$), 3.87 (2H, m, $CH_2OC$), 3.60 (2H, d, $CH_2Cl$), 2.31-1.96 (2H, m, $CH_2P$), 1.35 (3H, t, $CH_3$), 1.28 (6H, t, $2xCH_3$).

Eine Lösung von 7.2 g 3-Chlor-2(R)-hydroxy-propyl-(diethoxymethyl)phosphinsäureethylester, 3.6 g Hünig'scher Base und 6.5 g 3,4-Dichlorbenzylamin in 20 ml wasserfreiem Ethanol wird 4 Tage zum Rückfluss erhitzt. Man dampft zur Trockne ein und verteilt den Rückstand zwischen Dichlormethan und Wasser. Die organische Phase wird abgetrennt, über Natriumsulfat getrocknet, eingedampft und an Silicagel chromatographische gereinigt. Man erhält 3-[N-(3,4-Dichlorbenzyl)amino]-2(S)-hydroxy-propyl(diethoxymethyl)phosphinsäureethylester als fahlgelbes Öl; $^1$H-NMR ($CD_3Cl_3/D_2O$): δ= 7.43 (1H, m, Phenyl), 7.39 (1H, m. Phenyl), 7.15 (1H, m, Phenyl), 4.70 (1H, s, CHP), 4.20 (3H, m, CHO+$CH_2$OP), 3.83 (2H, m, $CH_2$OC), 3.70 (4H, m, $CH_2$Phenyl+$CH_2$OC), 2.65 (2H, m, $CH_2$N), 2.20-1.71 (2H, m, $CH_2$P), 1.40-1.26 (6H, m, 3x$CH_3$).

Beispiel 33: In analoger Weise wie in Beispiel 2 beschrieben kann man 3-{N-[1-(4-Chlor-3-jod-phenyl)ethyl]amino)-2(S)-hydroxy-propyl(diethoxymethyl)- phosphinsäure herstellen; $^1$H-NMR ($CD_3OD$): δ=8.10 (1H, d), 7.58 (2H, m), 4.45-4.18 (3H, m), 3.85 (4H, m), 2.98 (2H, $AB_2$, d), 2.10-1.52 (5H, m).

Beispiel 34: In analoger Weise wie in den Beispielen 1 bis 5, 7 und 11 bis 29 beschrieben kann man auch die nachstehend aufgeführten Verbindungen herstellen:

3-{N-[1(R)-(3,4-Dichlorphenyl)ethyl]amino}-2(S)-hydroxy-propyl-(cyclohex-3-enylmethyl)-phosphinsäure,

3-{N-[1(S)-(3,4-Dichlorphenyl)ethyl]amino}-2(S)-hydroxy-propyl-(cyclohex-3-enylmethyl)-phosphinsäure,

3-{N-[1(R)-(3,4-Dichlorphenyl)ethyl]amino}-2(R)-hydroxy-propyl-(cyclohex-3-enylmethyl)-phosphinsäure,

3-{N-[1(S)-(3,4-Dichlorphenyl)ethyl]amino}-2(S)-hydroxy-propyl-(benzyl)-phosphinsäure,

3-{N-[1(S)-(3,4-Dichlorphenyl)ethyl]amino}-2(S)-hydroxy-propyl-(diethoxymethyl)-phosphinsäure,

3-{N-[1(R)-(3,4-Dichlorphenyl)ethyl]amino}-2(S)-hydroxy-propyl-(diethoxymethyl)-phosphinsäure,

3-{N-[3-(3,4-Dichlorphenyl)propyl]amino}-2(S)-hydroxy-propyl-(benzyl)-phosphinsäure,

3-{N-[3(R)-(Phenyl)prop-2(R)-yl]amino}-2(S)-hydroxy-propyl-(benzyl)-phosphinsäure,

3-{N-[3(R)-(Phenyl)prop-2(R)-yl]amino}-2(S)-hydroxy-propyl-(cyclohexylmethyl)-phosphinsäure,

3-{N-[3(R)-(Phenyl)-3-hydroxy-prop-2(R)-yl]amino}-2(S)-hydroxy-propyl-(benzyl)-phosphinsäure,

3-{N-[3(R)-(Phenyl)-3-hydroxy-prop-2(R)-yl]amino}-2(S)-hydroxy-propyl-(cyclohexylmethyl)-phosphinsäure,

3-{N-[3-(3,4-Dichlorphenyl)-3-hydroxy-prop-2-yl]amino}-2(S)-hydroxy-propyl(benzyl)-phosphinsäure,

3-{N-[2-(3,4-Dichlorphenyl)propyl]amino}-2( S)-hydroxy-propyl-(cyclohexylmethyl)-phosphinsäure,

3-{N-[2-(3,4-Dichlorphenyl)propyl]amino}-2( S)-hydroxy-propyl-(benzyl)-phosphinsäure,

3-{N-[3-(3,4-Dichlorphenyl)prop-2-yl]amino}-2(S)-hydroxy-propyl-(benzyl)-phosphinsäure,

3-{N-[4-(3,4-Dichlorphenyl)but-3-yl]amino}-2( S)-hydroxy-propyl-(benzyl)-phosphinsäure,

3-{N-[1-(3,4-Dichlorphenyl)-2-hydroxy-ethyl]amino}-2(S)-hydroxy-propyl-(benzyl)-phosphinsäure,

3-{N-[2-(3,4-Dichlorphenyl)-2-hydroxy-ethyl]amino}-2(S)-hydroxy-propyl-(benzyl)-phosphinsäure,

3-{N-[1(S)-(3-Chlorphenyl)ethyl]amino}propyl-(cyclohexylmethyl)-phosphinsäure und

3-{N-[1(S)-(3-Chlorphenyl)ethyl]amino}-2( S)-hydroxy-propyl-(cyclohexylmethyl)-phosphinsäure.

Beispiel 35: Zu einer Lösung von 1.1 g 3-Amino-2(S)-hydroxy-propyl-(cyclohex-3-enyl)-phosphinsäureethylester in 4 ml Ethanol werden 0.2 g Lithiumhydroxid und 4 ml Wasser zugegeben. Man erhitzt auf 60° und lässt 20 Stunden rühren. Dann lässt man auf Raumtemperatur abkühlen, stellt mit wässriger Phosphorsäure auf pH 7 ein, dampft zur Trockne ein, nimmt in Methanol/Wasser auf und filtriert. Nach Abziehen des Lösungsmittels hinterbleibt ein Schaum, der nach Kristallisation aus Ethanol/Methanol 3-Amino-2(S)-hydroxy-propyl-(cyclohex-3-enyl)-phosphinsäure in Form weisser Kristalle vom Smp. 221-225° ergibt.

Das Ausgangsmaterial kann z.B. folgendermassen hergestellt werden:

Man Löst 8.7 g Natriumhydrid (99 %-ig) in 600 ml Tetrahydrofuran wird unter Stickstoff auf 15° gekühlt und

innerhalb 1 Stunde mit 75 g 1,1-Diethoxyethylphosphinsäureethylester in 100 ml Tetrahydrofuran versetzt, wobei die Reaktionstemperatur unter 25° gehalten wird. Die erhaltene Suspension wird 2 Stunden bei Raumtemperatur gerührt und dann innerhalb von 30 Minuten mit einer Lösung von 57.2 g Brommethylcyclohex-3-en in 100 ml wasserfreiem Tetrahydrofuran versetzt. Man erhitzt 24 Stunden zum Rückfluss, kühlt auf 0°, fügt 10 ml Wasser hinzu und zieht das Lösungsmittel ab. Der Rückstand wird in Dichlormethan gelöst, zweimal mit Wasser gewaschen, über Natriumsulfat getrocknet, zur Trockne eingedampft und destilliert. Der erhaltene 1,1-Diethoxyethyl-(cyclohex-3-en)-phosphinsäureethylester wird in 300 ml Dichlormethan, enthaltend 10 Vol-% Ethanol, gelöst und 24 Stunden bei Raumtemperatur stehengelassen. Nach Abziehen des Lösungsmittels und Destillation erhält man Cyclohex-3-enylmethylphosphinsäureethylester; $^{31}P$-NMR ($CDCl_3$): $\delta$ = 37.1 (s).

Eine Lösung von 16.8 g Cyclohex-3-enylmethylphosphinsäureethylester und 9.9 g Triethylamin in 120 ml Tetrahydrofuran wird mit 10.6 g Trimethylchlorsilan versetzt und 24 Stunden bei Raumtemperatur gerührt. Dann wird unter Argon filtriert und zur Trockne eingedampft. Das zurückbleibende Öl wird mit 9.1 g (R)-Epichlorhydrin und 1.1 g wasserfreiem Zinkchlorid versetzt. Es setzt eine exotherme Reaktion ein, nach deren Abklingen man 16 Stunden bei 80° nachrühren lässt. Die Reaktionsmischung wird auf Raumtemperatur abgekühlt, mit Dichlormethan verdünnt und mit Wasser gewaschen. Die organische Phase wird abgetrennt, über Natriumsulfat getrocknet und zur Trockne eingedampft. Das zurückbleibende farblose Öl wird in 250 ml Methanol, enthaltend 1 Vol-% Eisessig, gelöst und 24 Stunden bei Raumtemperaturstehengelassen. Nach Abziehen des Lösungsmittels und Chromatographie des Rückstandes an Silicagel erhält man 3-Chlor-2(S)-hydroxy-propyl-(cyclohex-3-enyl)-phosphinsäureethylester; $[\alpha]^{D}_{20}$ = +10.0 ± 1.2 (c= 0.84 in Trichlormethan).

Eine Lösung von 2.0 g 3-Chlor-2(S)-hydroxy-propyl-(cyclohex-3-enyl)-phosphinsäureethylester in 40 ml Ethanol wird in einen Autoklaven überführt. Man presst 10.0 g Ammoniak auf, lässt 72 Stunden bei Raumtemperatur stehen, dampft zur Trockne ein, chromatographiert an Silicagel, verrührt bei 0° mit Essigsäureethylester/Diethylether, und saugt ab. Man erhält 3-Amino-2(S)-hydroxy-propyl-(cyclohex-3-enyl)-phosphinsäureethylester, $[\alpha]^{D}_{20}$ = -5.2 ± 0.9 (c= 1.07 in Methanol).

Beispiel 36: In analoger Weise wie in Beispiel 35 beschrieben kann man 3-Amino-2(S)-hydroxy-propyl-(4-chlorbenzyl)-phosphinsäure-hydrochlorid, Smp. 158-162° herstellen.

Beispiel 37: In analoger Weise wie in Beispiel 35 beschrieben kann man 3-Amino-2(S)-hydroxy-propyl-(4-methylbenzyl)-phosphinsäure-hydrochlorid, Smp. 174-176° herstellen.

Beispiel 38: In analoger Weise wie in Beispiel 35 beschrieben kann man 3-Amino-2(S)-hydroxy-propyl-(4-methoxybenzyl)-phosphinsäure herstellen.

Beispiel 39: Tabletten, enthaltend je 200 mg 3-{N-1-(3,4-Dichlorphenyl)-ethyl]amino}-2(S)-hydroxy-propyl(cyclohexylmethyl)phosphinsäure oder ein Salz, z.B. das Hydrochlorid, davon können wie folgt hergestellt werden:

| Zusammensetzung (10000 Tabletten) | |
|---|---|
| Wirkstoff | 2000,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid (hochdispers) | 20,0 g |
| Ethanol | q.s. |

Der Wirkstoff wird mit der Lactose und 292 g Kartoffelstärke vermischt, die Mischung mit einer ethanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 295,0 mg Gewicht und 200,0 mg Wirkstoffgehalt, die gewünschtenfalls mit Teilkerben zur feineren Anpassung der Dosierung versehen sein können.

Beispiel 40: Lacktabletten, enthaltend je 400 mg 3-{N-1-(3,4-Dichlorphenyl)-ethyl]amino}-2(S)-hydroxy-propyl(cyclohexylmethyl)phosphinsäure oder ein Salz, z.B. das Hydrochlorid, davon können wie folgt hergestellt werden:

| Zusammensetzung (für 1000 Lacktabletten) | |
|---|---|
| Wirkstoff | 400,0 g |
| Lactose | 100,0 g |
| Maisstärke | 70,0 g |
| Talk | 8,5 g |
| Calciumstearat | 1,5 g |
| Hydroxypropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten mg) verpresst und diese mit einer Lösung der Hydroxypropyl-methylcellulose und des Schellacks in Methylenchlorid lackiert; Endgewicht der Lacktablette: 580mg.

Beispiel 41: Gelatinesteckkapseln, enthaltend 500 mg Wirkstoff, z.B. 3-{N-1-(3,4-Dichlorphenyl)ethyl]amino}-2(S)-hydroxy-propyl(cyclohexylmethyl)phosphinsäure oder ein Salz, z.B. das Hydrochlorid, davon, können z.B. folgendermassen hergestellt werden:

| Zusammensetzung (für 1000 Kapseln) | |
|---|---|
| Wirkstoff | 500,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Maschenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt wird das Magnesiumstearat durch ein Sieb mit einer Maschenweite von 0,8 mm hinzugesiebt. Nach 3- minütigem weiteren Mischen werden je 790 mg der erhaltenen Formulierung in Gelatinesteckkapseln passender Grösse abgefüllt.

Beispiel 42: Eine 5%-ige Injektions- oder Infusionslösung von 3-{N-1-(3,4-Dichlorphenyl)ethyl]amino}-2(S)-hydroxy-propyl(cyclohexylmethyl)phosphinsäure oder eines Salzes, z.B. des Hydrochlorides, davon, kann beispielsweise folgendermassen hergestellt werden:

| Zusammensetzung (für 1000 bzw. 400 Ampullen) | |
|---|---|
| Wirkstoff | 125,0 g |
| Natriumchlorid | 22,5 g |
| Phosphatpuffer pH= 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 50 bzw. 125 mg Wirkstoff enthalten.

**Patentansprüche**

**1.** Eine Verbindung der Formel I

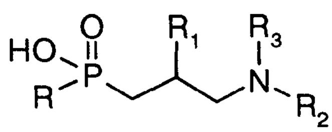

(I),

worin

a) R Diethoxymethyl, $R_1$ Wasserstoff und $R_2$ 2,6- oder 3,5-Dichlorbenzyl, Pyrid-3-ylmethyl, 1-(4-Methoxyphenyl)ethyl, 1-(4-Chlor-3-jod-phenyl)ethyl oder 1-(3-Chlor-4-jod-phenyl)ethyl oder $R_1$ Hydroxy und $R_2$ 3,4-Dichlorbenzyl, 1-(3-Chlor-4-jod-phenyl)ethyl, 1-(4-Chlor-3-jod-phenyl)ethyl oder 1-(3,4-Dichlorphenyl)ethyl und $R_3$ Wasserstoff bedeutet oder
b) R Cyclohexylmethyl, $R_1$ Wasserstoff und $R_2$ 3,5-Dichlorbenzyl, Chinolin-4-yl-methyl, 1-(3-Chlorphenyl)ethyl, oder 1-(3,4,5-Trimethoxyphenyl)ethyl oder $R_1$ Hydroxy und $R_2$ 3,4-Dimethylbenzyl, 3,4-Methylendioxybenzyl, 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,5-Dimethoxy-phenyl)ethyl, 1-(3,4,5-Trimethoxyphenyl)ethyl,3-Phenylprop-2-yl, 2-(3,4-Dichlorphenyl)propyl 2-(3,4-Dichlorphenyl)propyl, 3-(3,4-Dichlorphenyl)prop-2-yl oder 3-Phenyl-3-hydroxy-prop-2-yl und $R_3$ Wasserstoff bedeutet oder R Cyclohexylmethyl, $R_1$ Wasserstoff, $R_2$ 4-Chlorbenzyl und $R_3$ Methyl bedeutet oder
c) R Cylohex-3-enylmethyl, $R_1$ (S)-Hydroxy, $R_2$ 1(S)-(3,4-Dichlorphenyl)ethyl und $R_3$ Wasserstoff bedeutet oder
d) R Benzyl, $R_1$ Hydroxy, $R_2$ $\alpha$-Cyclopropyl-3,4-dichlor-benzyl, 3,4,5-Trimethoxybenzyl, 1-(3,5-Dimethoxyphenyl)ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,4,5-Trimethoxyphenyl)ethyl, 3-Phenylprop-2-yl, 3-Phenyl-3-hydroxy-prop-2-yl, 1-, 2- oder 3-(3,4-Dichlorphenyl)propyl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet, oder
e) R 4-Chlorbenzyl, 4-Methylbenzyl, 4-Methoxybenzyl oder Cyclohex-3-enmethyl bedeutet und $R_1$, $R_2$ und $R_3$ Wasserstoff darstellen,

oder ein Salz davon.

**2.** Eine Verbindung gemäss Anspruch 1 der Formel I,
worin

a) R Diethoxymethyl, $R_1$ Wasserstoff und $R_2$ 2,6- oder 3,5-Dichlorbenzyl, Pyrid-3-ylmethyl oder 1-(4-Methoxy)phenethyl oder $R_1$ Hydroxy und $R_2$ 3,4-Dichlorbenzyl, 1-(3-Chlor-4-jod-phenyl)ethyl oder 1-(4-Chlor-3-jod-phenyl)ethyl und $R_3$ Wasserstoff bedeutet oder

b) R Cyclohexylmethyl, $R_1$ Wasserstoff und $R_2$ 3,5-Dichlorbenzyl, 1-(3-Chlorphenyl)ethyl oder Chinolin-4-ylmethyl oder $R_1$ Hydroxy und $R_2$ 3,4-Dimethylbenzyl oder 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(3,5-Dimethoxyphenyl)ethyl, 1-(3,4,5-Trimethoxyphenyl)ethyl, 3-Phenylprop-2-yl, 3-(3,4-Dichlorphenyl)prop-2-yl oder 3-Phenyl-3-hydrory-prop-2-yl und $R_3$ Wasserstoff bedeutet oder $R_1$ Wasserstoff, $R_2$ 4-Chlorbenzyl und $R_3$ Methyl bedeutet oder

c) R Cylohex-3-enylmethyl, $R_1$ Hydroxy und $R_2$ 1-(3,4-Dichlorphenyl)ethyl und $R_3$ Wasserstoff bedeutet oder

d) R Benzyl, $R_1$ Hydroxy, $R_2$ 3,4,5-Trimethoxybenzyl, 1-(3,5-Dimethoxyphenyl)-ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-, 2- oder 3-(3,4-Dichlorphenyl)propyl, 3-Phenylprop-2-yl, 3-Phenyl-3-hydroxy-prop-2-yl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet,

oder ein Salz davon.

**3.** Eine Verbindung gemäss Anspruch 1 der Formel I, worin R Cyclohexylmethyl oder Benzyl bedeutet, $R_1$ Hydroxy ist, $R_2$ für 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,5-Dimethoxyphenyl)ethyl oder 1-(3,4,5-Trimethoxy)phenethyl steht und $R_3$ Wasserstoff ist, oder ein Salz davon.

**4.** Eine Verbindung gemäss Anspruch 1 der Formel I, worin R Cyclohexylmethyl oder Benzyl, $R_1$ Hydroxy, $R_2$ 1-(3,4,5-Trimethoxyphenyl)ethyl und $R_3$ Wasserstoff ist, oder ein Salz davon.

**5.** Eine Verbindung gemäss einem der Ansprüche 1 bis 4, in denen $R_1$ Hydroxy bedeutet und das die Hydroxygruppe tragende C-Atom sowie, sofern vorhanden, das $\alpha$-C-Atom von 1-(3,4-Dichlor-, 3-Chlor-4-jod-, 4-Chlor-3-jod-, 2,4-Dimethoxy-, 2,5-Dimethoxy-, 2,4-Dimethoxy-, 3,4-Dimethoxy-, 3,5-Dimethoxy-, 3,4,5-Trimethoxy bzw. 3,4-Methylendioxyphenyl)ethyl, $\alpha$-Cyclopropyl-4,4-dichlorbenzyl bzw. 1-(3,4-Dichlorphenyl)propyl (S)-Konfiguration aufweisen, wobei R, $R_2$ und $R_3$ die jeweils angegebenen Bedeutungen haben.

**6.** 3-(3,5-Dichlorbenzylamino)propyl(diethoxymethyl)phosphinsäure;
3-{N-1-(3,4-Dichlorphenyl)ethyl]amino}-2(S)-hydroxy-propyl(cyclohexylmethyl)-phosphinsäure;
3-[N-(2,6-Dichlorbenzyl)amino]propyl-(diethoxymethyl)-phosphinsäure;
3-[N-(Pyrid-3-ylmethyl)amino]propyl-(diethoxymethyl)-phosphinsäure;
3-{N-[1-(4-Methoxyphenyl)ethyl]amino}propyl-(diethoxymethyl)- phosphinsäure;
3-[N-(3,4,5-Trimethoxybenzyl)amino]-2(S)-hydroxy-propyl-(benzyl)-phosphinsäure;
3-[N-[1-(3,4-Dichlorphenyl)ethylamino]-2(S)-hydroxy-propyl-(benzyl)-phosphinsäure;
3-{N-1-(3,4-Dichlorphenyl)ethyl]amino]-2(S)-hydroxy-propyl-(cyclohex-3-enyl-methyl)-phosphinsäure;
3-[(3,5-Dichlorbenzyl)amino]-propyl-(cyclohexylmethyl)-phosphinsäure;
3-[N-(Chinolin-4-yl)methylamino]-propyl-(cyclohexylmethyl)-phosphinsäure;
3-[N-(4-Chlorbenzyl)-N-methyl-amino]-propyl-(cyclohexylmethyl)-phosphinsäure;
3-{N-[1-(3-Chlor-4-jod-phenyl)ethyl]amino}-2(S)-hydroxy-propyl-(cyclohexylmethyl)-phosphinsäure;
3-{N-[1-(4-Chlor-3-jod-phenyl)ethyl]amino}-2(S)-hydroxy-propyl-(cyclohexylmethyl)-phosphinsäure;
3-{N-[1-(4-Chlor-3-jod-phenyl)ethyl]amino}propyl-(diethoxymet hyl)-phosphinsäure;
3-{N-[1(S)-(3,4-Dichlorphenyl)ethyl]amino}-2(S)-hydroxy-propyl-(cyclohexylmethyl)-phosphinsäure;
3-{N-[1(S)-(3,4-Dichlorphenyl)ethyl]amino}-2(R)-hydroxy-propyl-(cyclohexylmethyl)-phosphinsäure;
3-{N-[1(R)-(3,4-Dichlorphenyl)ethyl]amino}-2(R)-hydroxy-propyl-(cyclohexylmethyl)-phosphinsäure;
3-{N-[1(R)-(3,4-Dichlorphenyl)ethyl]amino}-2(S)-hydroxy-propyl-(cyclohexylmethyl)-phosphinsäure;
3-{N-[1-(3-Chlor-4-jod-phenyl)ethyl]amino}-2(S)-hydroxy-propyl-(benzyl)-phosphinsäure;
3-{N-[1-(3-Chlor-4-jod-phenyl)ethyl]amino}-2(S)-hydroxy-propyl-(benzyl)-phosphinsäure oder
3-{N-[1-(3-Chlor-4-jod-phenyl)ethyl]amino}propyl-(diethoxymet hyl)-phosphinsäure oder jeweils ein Salz davon.

**7.** 3-{N-[1-(3,4,5-Trimethoxyphenyl)ethylamino)-2(S)-hydroxy-propyl-cyclohexylmethyl-phosphinsäure oder ein Salz davon.

**8.** 3-{N-[1(S)-(3,4,5-Trimethoxyphenyl)ethylamino)-2(S)-hydroxy-propyl-cyclohexylmethyl-phosphinsäure oder ein Salz davon.

**9.** 3-{N-[1-(3,4-Methylendioxyphenyl)ethyl]amino}-2(S)-hydroxy-propyl-(benzyl)-phosphinsäure oder ein Salz davon.

**10.** 3-{N-[1-(3,4-Dimethoxyphenyl)ethylamino)-2(S)-hydroxy-propyl-(benzyl)-phosphinsäure oder ein Salz davon.

**11.** 3-{N-[1-(3,4-Dimethoxyphenyl)ethylamino}-2(S)-hydroxy-propyl-(cyclohexylmethyl)-phosphinsäure oder ein Salz davon.

**12.** 3-{N-[1-(3,4-Methylendioxyphenyl)ethylamino}-2(S)-hydroxy-propyl-(cyclohexylmethyl)-phosphinsäure oder ein Salz davon.

**13.** 3-[N-(3,4-Methylendioxybenzyl)amino]2(S)-hydroxy-propyl-(cyclohexylmethyl)-phosphinsäure oder ein Salz davon.

**14.** 3-{N-[1(S)-(3,4,5-Trimethoxyphenyl)ethyl]amino}-2(S)-hydroxy-propyl-(benzyl)-phosphinsäure oder ein Salz davon.

**15.** 3-{N-[1(S)-(3,4,5-Trimethoxyphenyl)ethyl]amino}-propyl-2(S)-hydroxy-(cyclohexylmethyl)-phosphinsäure oder ein Salz davon.

**16.** 3-{N-[1-(3,4-Dichlorphenyl)ethyl]amino}-2(S)-hydroxy-propyl-(cyclohexylmethyl)-phosphinsäure;
3-{N-[1-(3,5-Dimethoxyphenyl)ethyl]amino}-2(S)-hydroxy-propyl-(benzyl)-phosphinsäure;
3-[N-(3,4-Dichloro-αcyclopropyl-benzyl)amino]-2(S)-hydroxy-propyl-(benzyl)-phosphinsäure;
3-{N-[1(R)-(3,4-Dichlorphenyl)ethylamino]-2(S)-hydroxy-propyl-(benzyl)-phosphinsäure;
3-[N-(3,4-Dimethylbenzyl)amino]-2(S)-hydroxy-propyl-(cyclohexylmethyl)-phosphinsäure;
3-{N-[1-(2,5-Dimethoxyphenyl)ethyl]amino}-2(S)-hydroxy-propyl-(benzyl)-phosphinsäure;
3-{N-[1-(2,6-Dimethoxyphenyl)ethyl]amino}-2(S)-hydroxy-propyl-(benzyl)-phosphinsäure;
3-{N-[1-(3,4-Methylendioxyphenyl)ethyl]amino}-2(S)-hydroxy-propyl-(benzyl)-phosphinsäure;
3-{N-[1-(3,4-Dimethoxyphenyl)ethyl]amino}-2(S)-hydroxy-propyl-(benzyl)-phosphinsäure;
3-{N-[1-(2,4-Dimethoxyphenyl)ethyl]amino}-2(S)-hydroxy-propyl-(benzyl)-phosphinsäure;
3-{N-[1-(2,5-Dimethoxyphenyl)ethylamino}-2(S)-hydroxy-propyl-(cyclohexylmethyl)-phosphinsäure;
3-{N-[1-(2,6-Dimethoxyphenyl)ethylamino)-2(S)-hydroxy-propyl-(cyclohexylmethyl)-phosphinsäure;
3-{N-[1-(3,4-Dimethoxyphenyl)ethylamino}-2(S)-hydroxy-propyl-(cyclohexylmethyl)-phosphinsäure;
3-{N-[1-(2,4-Dimethoxyphenyl)ethylamino}-2(S)-hydroxy-propyl-(cyclohexylmethyl)-phosphinsäure;
3-{N-[1-(3,4-Methylendioxyphenyl)ethylamino}-2(S)-hydroxy-propyl-(cyclohexylmethyl)-phosphinsäure;
3-[N-(3,4-Methylendioxybenzyl)amino]-2(S)-hydroxy-propyl-(cyclohexylmethyl)-phosphinsäure;
3-[N-(3,4-Dichlorbenzyl)amino]-2(S)-hydroxy-propyl-(diethoxymethyl)phosphinsäure;
3-Amino-2(S)-hydroxy-propyl-(cyclohex-3-enyl)-phosphinsäure;
3-Amino-2(S)-hydroxy-propyl-(4-chlorbenzyl)-phosphinsäure;
3-Amino-2(S)-hydroxy-propyl-(4-methylbenzyl)-phosphinsäure oder
3-Amino-2(S)-hydroxy-propyl-(4-methoxybenzyl)-phosphinsäure oder jeweils ein Salz davon.

**17.** 3-{N-[1-(3,5-Dimethoxyphenyl)ethyl]amino}-2(S)-hydroxy-propyl-(cyclohexylmethyl)-phosphinsäure;
3-{N-[1(R)-(3,4-Dichlorphenyl)ethyl]amino}-2(S)-hydroxy-propyl-(cyclohex-3-enyl-methyl)-phosphinsäure;
3-{N-[1(S)-(3,4-Dichlorphenyl)ethyl]amino}-2(S)-hydroxy-propyl-(cyclohex-3-enyl-methyl)-phosphinsäure;
3-{N-[1(R)-(3,4-Dichlorphenyl)ethyl]amino}-2(R)-hydroxy-propyl-(cyclohex-3-enyl-methyl)-phosphinsäure;
3-{N-[1(S)-(3,4-Dichlorphenyl)ethyl]amino}-2(R)-hydroxy-propyl-(benzyl)-phosphinsäure;
3-{N-[1(R)-(3,4-Dichlorphenyl)ethyl]amino}-2(S)-hydroxy-propyl-(benzyl)-phosphinsäure;
3-{N-[1(S)-(3,4-Dichlorphenyl)ethyl]amino}-2(S)-hydroxy-propyl-(diethoxymethyl)-phosphinsäure;
3-{N-[1(R)-(3,4-Dichlorphenyl)ethyl]amino}-2(S)-hydroxy-propyl-(diethoxymethyl)-phosphinsäure;
3-{N-[3-(3,4-Dichlorphenyl)propyl]amino}-2(S)-hydroxy-propyl-(benzyl)-phosphinsäure;
3-{N-[3(R)-(Phenyl)prop-2(R)-yl]amino]-2(S)-hydroxy-propyl-(benzyl)-phosphinsäure;
3-(N-[3(R)-(Phenyl)prop-2(R)-yl]amino)-2(S)-hydroxy-propyl-(cyclohexylmethyl)-phosphinsäure;
3-{N-[3(R)-(Phenyl)-3-hydroxy-prop-2(R)-yl]amino)-2(S)-hydroxy-propyl-(benzyl)-phosphinsäure;
3-{N-[3(R)-(Phenyl)-3-hydroxy-prop-2(R)-yl]amino}-2(S)-hydroxy-propyl-(cyclohexylmethyl)-phosphinsäure;
3-{N-[3-(3,4-Dichlorphenyl)-3-hydroxy-prop-2-yl]amino}-2(S)-hydroxy-propyl-(benzyl)-phosphinsäure;
3-{N-[2-(3,4-Dichlorphenyl)propyl]amino}-2( S)-hydroxy-propyl-(cyclohexylmethyl)-phosphinsäure;
3-{N-[2-(3,4-Dichlorphenyl)propyl]amino}-2( S)-hydroxy-propyl-(benzyl)-phosphinsäure,
3-{N-[3-(3,4-Dichlorphenyl)prop-2-yl]amino}-2(S)-hydroxy-propyl-(benzyl)-phosphinsäure;
3-{N-[4-(3,4-Dichlorphenyl)but-3-yl]amino}-2( S)-hydroxy-propyl-(benzyl)-phosphinsäure;
3-{N-[1-(3,4-Dichlorphenyl)-2-hydroxy-ethyl]amino}-2(S)-hydroxy-propyl-(benzyl)-phosphinsäure;

3-{N-[2-(3,4-Dichlorphenyl)-2-hydroxy-ethyl]amino}-2(S)-hydroxy-propyl-(benzyl)-phosphinsäure;
3-{N-[1(S)-(3-Chlorphenyl)ethyl]amino}propyl-(cyclohexylmethyl)-phosphinsäure oder
3-{N-[1(S)-(3-Chlorphenyl)ethyl]amino}-2( S)-hydroxy-propyl(cyclohexylmethyl)-phosphinsäure oder jeweils ein Salz davon.

**18.** Eine Verbindung gemäss einem der Ansprüche 1, 3 bis 5 und 7 bis 16 zur Behandlung des menschlichen oder tierischen Körpers.

**19.** Eine Verbindung gemäss einem der Ansprüche 2, 6 und 17 zur Behandlung des menschlichen oder tierischen Körpers.

**20.** Pharmazeutische Präparate, enthaltend eine Verbindung gemäss einem der Ansprüche 1, 3 bis 5, 7 bis 16 und 18 neben üblichen pharmazeutischen Hilfs- und Trägerstoffen.

**21.** Pharmazeutische Präparate, enthaltend eine Verbindung gemäss einem der Ansprüche 2, 6, 17 und 19 neben üblichen pharmazeutischen Hilfs- und Trägerstoffen.

**22.** Verfahren zur Herstellung von Verbindungen der Formel I

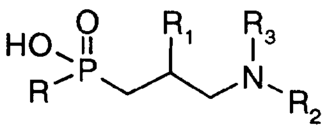

(I),

worin

a) R Diethoxymethyl, $R_1$ Wasserstoff und $R_2$ 2,6- oder 3,5-Dichlorbenzyl, Pyrid-3-ylmethyl, 1-(4-Methoxyphenyl)ethyl, 1-(4-Chlor-3-jod-phenyl)ethyl oder 1-(3-Chlor-4-jod-phenyl)ethyl oder $R_1$ Hydroxy und $R_2$ 3,4-Dichlorbenzyl, 1-(3-Chlor-4-jod-phenyl)ethyl, 1-(4-Chlor-3-jod-phenyl)ethyl oder 1-(3,4-Dichlorphenyl)ethyl und $R_3$ Wasserstoff bedeutet oder
b) R Cyclohexylmethyl, $R_1$ Wasserstoff und $R_2$ 3,5-Dichlorbenzyl, Chinolin-4-ylmethyl, 1-(3-Chlorphenyl)ethyl, oder 1-(3,4,5-Trimethoxyphenyl)ethyl oder $R_1$ Hydroxy und $R_2$ 3,4-Dimethylbenzyl, 3,4-Methylendioxybenzyl, 1-(3-Chlorphenyl)ethyl, 1-(3,4-Dichlorphenyl)ethyl, 1-(3-Chlor-4-jod-phenyl)ethyl, 1-(4-Chlor-3-jod-phenyl)ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,5-Dimethoxy-phenyl)ethyl, 1-(3,4,5-Trimethoxy)phenethyl,3-Phenylprop-2-yl, 2-(3,4-Dichlorphenyl)propyl 2-(3,4-Dichlorphenyl)propyl, 3-(3,4-Dichlorphenyl)prop-2-yl oder 3-Phenyl-3-hydroxy-prop-2-yl und $R_3$ Wasserstoff bedeutet oder R Cyclohexylmethyl, $R_1$ Wasserstoff, $R_2$ 4-Chlorbenzyl und $R_3$ Methyl bedeutet oder
c) R Cylohex-3-enylmethyl, $R_1$ (S)-Hydroxy, $R_2$ 1(S)-(3,4-Dichlorphenyl)ethyl und $R_3$ Wasserstoff bedeutet oder
d) R Benzyl, $R_1$ Hydroxy, $R_2$ $\alpha$-Cyclopropyl-3,4-dichlor-benzyl, 3,4,5-Trimethoxybenzyl, 1-(3,5-Dimethoxyphenyl)ethyl, 1-(3,4-Dichlorphenyl)ethyl, 2-(3,4-Dichlorphenyl)ethyl, 1-(3-Chlor-4-jod-phenyl)ethyl, 1-(3,4-Dichlorphenyl)-2-hydroxy-ethyl, 2-(3,4-Dichlorphenyl)-2-hydroxy-ethyl, 1-(2,4-Dimethoxyphenyl)ethyl, 1-(2,5-Dimethoxyphenyl)ethyl, 1-(2,6-Dimethoxyphenyl)ethyl, 1-(3,4-Dimethoxyphenyl)ethyl, 1-(3,4-Methylendioxyphenyl)ethyl, 1-(3,4,5-Trimethoxyphenyl)ethyl, 3-Phenylprop-2-yl, 3-Phenyl-3-hydroxy-prop-2-yl, 1-, 2- oder 3-(3,4-Dichlorphenyl)propyl, 3-(3,4-Dichlorphenyl)prop-2-yl, 3-(3,4-Dichlorphenyl)-3-hydroxy-prop-2-yl oder 4-(3,4-Dichlorphenyl)butyl und $R_3$ Wasserstoff bedeutet, oder
e) R 4-Chlorbenzyl, 4-Methylbenzyl, 4-Methoxybenzyl oder Cyclohex-3-enmethyl bedeutet und $R_1$, $R_2$ und $R_3$ Wasserstoff darstellen,

und ihrer Salze, dadurch gekennzeichnet, dass man in einer Verbindung der Formel II

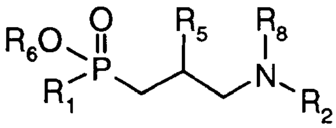

(II),

worin für Wasserstoff oder geschütztes Hydroxy steht, $R_6$ für eine Hydroxyschutzgruppe, $R_8$ für eine Gruppe $R_3$ oder für eine Aminoschutzgruppe und R, R die angegebene Bedeutung hat, oder in einem Salz davon die Hydroxygruppen durch Ersatz der Hydroxyschutzgruppe $R_6$ durch Wasserstoff freisetzt und gegebenenfalls die Aminoschutzgruppe $R_8$ abspaltet und gegebenenfalls die Hydroxygruppen $R_1$ aus der geschützten Hydroxygruppe $R_5$ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt.

**23.** Verwendung einer Verbindung gemäss einem der Ansprüche 1 bis 19 zur Herstellung eines für die Behandlung von auf $GABA_B$-Antagonisten ansprechenden Erkrankungen bestimmten Arzneimittels.

Europäisches Patentamt

**EUROPÄISCHER TEILRECHERCHENBERICHT** der nach Regel 45 des Europäischen Patentübereinkommens für das weitere Verfahren als europäischer Recherchenbericht gilt

Nummer der Anmeldung: EP 96 11 8735

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.6) |
|---|---|---|---|
| Y | EP 0 319 482 A (CIBA-GEIGY AG)<br>* das ganze Dokument *<br>--- | 1-23 | C07F9/30<br>A61K31/66<br>C07F9/58<br>C07F9/60<br>C07F9/655 |
| Y | EP 0 356 128 A (SMITH KLINE & FRENCH)<br>* das ganze Dokument *<br>--- | 1-23 | |
| Y | EP 0 399 949 A (CIBA-GEIGY AG)<br>* das ganze Dokument *<br>--- | 1-23 | |
| Y | EP 0 402 312 A (CIBA-GEIGY AG)<br>* das ganze Dokument *<br>--- | 1-23 | |
| P,X | EP 0 463 560 A (CIBA-GEIGY AG)<br>* das ganze Dokument und insbesonders Seite 33, Zeile 11 und Seite 34, Zeile 7 *<br>----- | 1,2,6,<br>16,18-23 | |

| RECHERCHIERTE SACHGEBIETE (Int.Cl.6) |
|---|
| C07F<br>A61K |

## UNVOLLSTÄNDIGE RECHERCHE

**Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.**
**Vollständig recherchierte Patentansprüche:**
**Unvollständig recherchierte Patentansprüche:**
**Nicht recherchierte Patentansprüche:**
**Grund für die Beschränkung der Recherche:**

Siehe Ergänzungsblatt C

| Recherchenort | Abschlußdatum der Recherche | Prüfer |
|---|---|---|
| DEN HAAG | 20.Januar 1997 | Beslier, L |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

Europäisches Patentamt

EP 96 11 8735 - C -

**UNVOLLSTÄNDIGE RECHERCHE**

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:
Unvollständig recherchierte Patentansprüche:
Nicht recherchierte Patentansprüche:

Grund für die Beschränkung der Recherche: Obwohl die Ansprüche 18,19 sich auf ein Verfahren zur Behandlung des menschlichen/tierischen Körpers beziehen (Artikel 52(4) EPÜ), wurde die Recherche durchgeführt und gründete sich auf die angeführten Wirkungen der Verbindungen.

EPO Form Supplementary Sheet C (1996)